# EUROPÄISCHE PATENTSCHRIFT

(11) **EP 3 241 567 B1**
(45) Veröffentlichungstag und Bekanntmachung des Hinweises auf die Patenterteilung: **22.04.2026**
(21) Anmeldenummer: 17169999.4
(22) Anmeldetag: 08.05.2017
(51) Int. Cl.: A61L 2/07

(54) **VERFAHREN ZUR STERILISATION VON HANDSCHUHEN**
METHOD FOR THE STERILISATION OF GLOVES
PROCÉDÉ DE STÉRILISATION DE GANTS

(30) Priorität: 06.05.2016 DE 102016108398
(43) Veröffentlichungstag der Anmeldung: 08.11.2017
(73) Patentinhaber: Frank, Gerald, 63303 Dreieich (DE)
(72) Erfinder: Frank, Gerald, 63303 Dreieich (DE)
(74) Vertreter: Tergau & Walkenhorst Intellectual Property GmbH

(56) Entgegenhaltungen:
- WO-A2-2009/072800
- CH-A1- 707 655
- DE-A1- 1 492 319
- DE-U1- 202010 010 331
- KR-B1- 101 497 060
- US-A- 2 340 206
- US-A- 3 166 439
- US-B1- 8 966 781

## Beschreibung

Die Erfindung betrifft ein Verfahren zur Sterilisation von Handschuhen nach dem Oberbegriff von Anspruch 1
Handschuhe, die z. B. in Abfüllanlagen für Parenteralia, welche nach der sogenannten RABS-Technologie (Restricted Access Barrier System) ausgelegt sind oder in Reinraum Klasse A Bereich eingesetzt werden sollen, müssen vor ihrer Verwendung sterilisiert werden. Dazu werden sie gewöhnlich in einer Dampfsterilisationskammer sterilisierendem Dampf ausgesetzt. Dabei ist eine kurze Sterilisierzeit sehr wichtig, um das Gummimaterial der Handschuhe nicht zu stark zu schädigen. Der Sterilisiererfolg ist allerdings nur dann sichergestellt, wenn der Dampf bis in die inneren Fingerspitzen eindringt. Es muss demnach Sorge dafür getragen werden, dass zum einen die in den Handschuhen befindliche Luft und /oder nichtkondensierbare Gase entfernt werden und zum anderen der Dampf in möglichst kurzer Zeit zu allen Kontaktflächen auch bis in die Fingerspitzen des Handschuhs vordringen bzw. diese auch erreichen kann. Es sind zu diesem Zweck Drahtgestelle bekannt, auf die ein Handschuh aufgezogen wird, bevor er in der Sterilisationskammer Dampf ausgesetzt wird.

Aus der DE 1 300 634 A ist eine Vorrichtung zur Vorbereitung von Gummihandschuhen für den medizinischen Gebrauch bekannt, welche ein der Handschuhform angepasstes hohles Handschuhgestell aufweist, auf das der zu sterilisierende Handschuh aufgezogen wird.

Die US 2 340 206 A beschreibt ein Verfahren zur Dampfsterilisation von Handschuhen, in welchem eine Einbringvorrichtung in den Handschuh eingebracht wird und der Handschuh mit eingebrachter Einbringvorrichtung in einer Dampfsterilisationskammer deponiert wird und daraufhin Sterilisationsdampf in die Kammer eingeleitet wird.

Aus der US 3 166 439 A ist eine Vorrichtung für Handschuhe bekannt, welche einen Grundkörper und Fingerkörper umfasst und auf welche der Handschuh während der Reinigung aufgespannt werden kann.

Aus der DE 14 92 319 A1ist eine Aufbereitungsanlage für Gummihandschuhe bekannt, bei welcher eine Station zum Aufziehen der Handschuhe auf ein Handschuhgestell vorgesehen ist.

Aus der DE 20 2010 010 331 U1 ist ein sterilisierbarer Polyolefinbeutel bekannt, dessen Material der eine dampfdurchlässige Lage und eine im Wesentlichen aus Polyolefin gebildete Folie aufweist. Der Behälter kann auch aus Handschuh ausgebildet sein.

Die US 8 966 781 B1 beschreibt ein Gestell mit mehreren Halterung für unterschiedliche Teile einer sportlichen Ausrüstung, die, beispielsweise mittels Dampf, gereinigt werden können, während sie auf dem Gestell angeordnet sind.

Aus der WO 2009/072800 A2 ist eine Vorrichtung zum Trocknen und Sterilisieren von Gummihandschuhen bekannt. Sie umfasst eine Fingerstütze für Handschuhe mit einem Fingerteil und einem Handgelenkteil, die in die Gummihandschuhe eingesetzt werden.

Die CH 707 655 A1 beschreibt eine Streckvorrichtung zum temporären Expandieren eines an einem Containment installierten Arbeitshandschuhs.

Aus der KR 101 497 060 B1 ist eine Desinfektionsvorrichtung für Gummihandschuhe mit einem Gehäuse bekannt mit einem Desinfektionsteil, das in das Gehäuse eingebaut ist und den eingesetzten Gummihandschuh desinfiziert. Dabei ist ein inneres und äußeres Wendeteil vorhanden, das jeweils an beiden Stirnseiten der eingesetzten Gummihandschuhe befestigt ist und es ermöglicht, die eine Seite und die andere Seite miteinander zu kreuzen.

Nachteilig bei den bekannten Verfahren zur Sterilisation von Handschuhen sind der hohe Platzbedarf und die zeitaufwändige Bestückung der Handschuhe mit den Drahtgestellen. Zukünftige Sterilisationsprozesse erfordern dagegen hohe Kapazitäten.

Der Erfindung liegt daher die Aufgabe zugrunde, ein Verfahren zur Dampfsterilisation dahingehend zu verbessern, dass die Kapazität erhöht werden kann bei gleichzeitiger Zuverlässigkeit der vollständigen Sterilisation.

Diese Aufgabe wird erfindungsgemäß gelöst mit den Merkmalen des Anspruchs 1.

Vorteilhafte Ausgestaltungen der Erfindung sind Gegenstand der Unteransprüche.

Die Erfindung geht von der Überlegung aus, dass die Kapazität bei der Dampfsterilisation von Handschuhen bei bekannten Verfahren durch zwei wesentliche Faktoren begrenzt wird. Aufgrund des begrenzten Volumens der Sterilisationskammer kann nur eine geringe Anzahl an Handschuhen auf Drahtgestellen in der Kammer angeordnet werden. Zudem dauert das Anordnen eines Handschuhes auf einem Drahtgestell relativ lange. Es wäre daher wünschenswert, den Sterilisationsvorgang platzsparender und mit verminderter Handhabungszeit zu gestalten.

Wie nunmehr erkannt wurde, kann eine Kapazitätserhöhung erzielt werden, indem eine Einlage bzw. Einbringvorrichtung, die im Wesentlichen die Innenform des Handschuhs aufweist, aus einem dampfdurchlässigen Material gebildet wird und für die Sterilisation in dem Handschuh eingebracht ist. Wie Untersuchungen des Anmelders gezeigt haben, erfolgt eine gesicherte und zuverlässige Sterilisation, wenn die Einlage in den Handschuh eingelegt ist und seine gesamte Innenkontur ausfüllt. Da die Einlage aus dampfdurchlässigem Material hergestellt ist, kann der Dampf den gesamten Innenbereich des Handschuhs ausfüllen und auch in die Fingerspitzen gelangen.

Das Verfahren umfasst die folgenden Schritte:
a) Bereitstellen eines Handschuhs;
b) Einbringen einer, insbesondere flexiblen, Einbringvorrichtung in den Handschuh, welche eine Kontur aufweist, die im Wesentlichen formkongruent zu einer inneren Kontur des Handschuhs ist und einen Grundkörper aufweist, der aus dampfdurchlässigem Material gefertigt bzw. gebildet ist;
c) Deponieren des Handschuhs mit eingebrachter Einbringvorrichtung in einer Dampfsterilisationskammer;
d) Einleiten von sterilisierendem Dampf in die Dampfsterilisationskammer, wobei die Einbringvorrichtung derart in den Handschuh eingebracht wird, dass sie im Wesentlichen die gesamte innere Kontur des Handschuhs einnimmt.

Vorzugsweise erfolgt in einem Schritt b2), der vor Schritt c) durchgeführt wird, das Falten des Handschuhs inklusive der Einbringvorrichtung gemäß, insbesondere gemäß einer geforderten Konfiguration. Die Konfiguration beschreibt dabei, wie oft und in welcher Aufeinanderlegung der gefalteten Teile der Handschuh gefaltet werden soll.

Mögliche Konfigurationen sind u. a. einfach und doppelt aufeinandergeklappte bzw. ineinander gestülpte Varianten.

Der zur Sterilisierung verwendete Dampf ist bevorzugt Sattdampf, bevorzugt mit einer Temperatur zwischen 120-135 °C. Der entsprechende Druck des Sattdampfes ergibt sich bevorzugt aus der Sattdampftabelle.

Der zur sterilisierende Handschuh besteht vorteilhafterweise im Wesentlichen aus Gummi, CSM, EPDM, gummiartigen Materialien oder verwandten Materialien. Die Handschuhe können je nach Ausführung Einmal- (Single Use) oder mehrfachverwendbar ausgelegt sein.

Beim Einbringen der Einbringvorrichtung in den Handschuh wird bevorzugt der Handschuh zumindest teilweise räumlich fixiert, während die Einbringvorrichtung in ihn hineingeschoben wird.

Erfindungsgemäß wird nach Einbringen der Einbringvorrichtung in den Handschuh der Handschuh wenigstens einmal gefaltet. Bevorzugt wird dabei jeweils zwischen die sich nach der Faltung berührenden Außenbereiche der Handschuhe jeweils eine Trennlage bzw. ein Trennelement gelegt bzw. eingefügt, um ein Verkleben der äußeren sich berührenden Handschuhbereiche bzw. -flächen zu verhindern. Auf diese Weise wird vermieden, dass sich die äußeren Handschuhflächen berühren. Durch die Trennlage wird erreicht, dass auch diese Flächen von dem sterilisierenden Dampf sicher erreicht werden können. Die jeweilige Trennlage ist bevorzugt aus dem gleichen Material gefertigt wie die Einbringvorrichtung. Insbesondere eignen sich dazu die bei der Einbringvorrichtung angegebenen bevorzugten Materialien.

Untersuchungen haben ergeben, dass auch im gefalteten Zustand, insbesondere auch für komplex gefaltete Konfigurationen, eine vollständige Dampfsterilisation gegeben ist, da es erst die Einbringvorrichtung ermöglicht, dass der sterilisierende Dampf auch die gesamte Innenfläche des Handschuhs erreicht. Dadurch wird eine Platzersparnis bei gleichzeitiger Sicherstellung der Sterilität erreicht, so dass eine größere Menge an Handschuhen nachweisbar sicher sterilisiert werden kann. Die Faltung kann in unterschiedlichen Konfigurationen erfolgen. Die Konfiguration beschreibt dabei, wie oft und in welcher Aufeinanderlegung der gefalteten Teile der Handschuh gefaltet werden soll. Mögliche Konfigurationen sind u. a. einfach und doppelt aufeinandergeklappte bzw. ineinander gestülpte Varianten.

Die Kapazität kann erhöht werden bei gleichzeitiger Zuverlässigkeit der vollständigen Sterilisation insbesondere auch durch das Zusammenfalten des Handschuhs inklusive der Einbringvorrichtung. Das heißt, während der Handschuh gefaltet wird, ist die Einbringvorrichtung in ihn vollständig eingebracht.

Das Material, aus dem die Einbringvorrichtung im Wesentlichen gefertigt ist, besteht erfindungsgemäß aus mindestens einlagigen, flexiblem, dampfdurchlässigen und durch Dampf, insbesondere Sattdampf, sterilisierbarem Kunststoffmaterial. Das Material ist bevorzugt derart feinporig gebildet bzw. strukturiert, dass die Moleküle des Dampfes durch sie kontrolliert hindurchdringen bzw. diffundieren können. Auf diese Weise kann der Dampf die Einbringvorrichtung durchdringen und die gesamte Innenfläche des zu sterilisierenden Handschuhs erreichen. Das Material ist insbesondere flexibel und nicht steif, insbesondere auch elastisch. Es unterscheidet sich dadurch insbesondere von bekannten Gestellen, insbesondere aus Holz oder Metall, auf die der zu sterilisierende Handschuh aufgesteckt wird. Dadurch, dass das Material flexibel ist, wird eine Beschädigung des zu sterilisierenden Handschuhs während des Einbringens verhindert.

Die Einbringvorrichtung ist erfindungsgemäß flexibel und bevorzugt flach gestaltet. Sie weist bevorzugt eine Dicke auf, die im Bereich zwischen 10 bis 300 µm liegt. Bei mehrlagigem Material werden die Lagen bevorzugt durch eine Siegelnaht miteinander verbunden bzw. verschweißt.

Das Kunststoffmaterial besteht bevorzugt aus thermisch behandeltem Fasermaterial und /oder Vliesstoff. Das Fasermaterial und/oder der Vliesstoff umfasst bevorzugt Fasern von Thermoplasten oder mikrofeinem Polytetraflourethylen und/oder aus deren Mischungen, welche, insbesondere thermisch, derart behandelt wurden, dass eine Dampfdurchlässigkeit, insbesondere gegenüber Sattdampf, gegeben ist. So lange Dampfdurchlässigkeit gegeben ist können die Einzellagen unterschiedliche Flächengewichte aufweisen.

Das Material bzw. Kunststoffmaterial umfasst bevorzugt poröse dampfdurchlässige Materialien aus Polyethylen, Polyolefinen und/oder aus deren Mischmaterialien (Polyblends), insbesondere ein Fasermaterial und/oder Vliesstoff, insbesondere mit thermisch behandelten Fasern.

Die Einbringvorrichtung ist bevorzugt aus Tyvek^{®} oder porösem Kunststoffmaterial auf Polyolefinbasis gefertigt bzw. das Material wird bevorzugt als Tyvek^{®} oder porösem Kunststoffmaterial auf Polyolefinbasis gewählt.

In einer ersten bevorzugten Ausführungsform ist die Einbringvorrichtung einlagig ausgebildet. Diese Lage weist die Innenkontur des zu sterilisierenden Handschuhs auf.

Um das Einbringen der Einbringvorrichtung in den zu sterilisierenden Handschuh zu erleichtern, weist die Einbringvorrichtung bevorzugt Einführungselemente bzw. Führungselemente auf, die in die Finger des Handschuhs eingeführt werden. Das jeweilige Einführungselement ist bevorzugt jeweils an einer Fingerspitze der Kontur der Einbringvorrichtung angebracht, so dass bevorzugt insgesamt fünf Einführungselemente vorgesehen sind. Das jeweilige Einführungselement ist vorteilhafterweise in der Art eines Fingerhutes ausgebildet. Zum Einbringen der Einbringvorrichtung in den Handschuh kann der Benutzer mit seinen Fingerspitzen jeweils ein Einführungselement aufnehmen und seine Fingerspitze in die Spitze des jeweiligen Handschuhfingers bewegen. Auf diese Weise wird zuverlässig erreicht, dass die Einbringvorrichtung die gesamte Innenkontur des Handschuhs abdeckt bzw. mit ihr räumlich überlappt.

In einer zweiten bevorzugten Ausführungsform ist die Einbringvorrichtung zumindest zweilagig ausgebildet, wobei jede Lage mit zumindest einer weiteren Lage zumindest teilweise an einem Randbereich miteinander verbunden ist.

Die Einbringvorrichtung ist dabei bevorzugt in der Form eines Handschuhs ausgebildet.

Vorteilhafterweise ist die Einbringvorrichtung zweilagig ausgebildet und die beiden Lagen sind derart miteinander verbunden, dass sie einen Handschuh bilden. Eine derartige Ausbildung erlaubt eine komfortable Einführung in den zu sterilisierenden Handschuh. Der Benutzer streift sich die Einbringvorrichtung wie einen Handschuh über und streift sich dann den zu sterilisierenden Handschuh über. Die Einbringvorrichtung fungiert dabei gewissermaßen als Innenhandschuh für den zu sterilisierenden Handschuh. Es kann durch diesen Überziehvorgang auch sichergestellt werden, dass die Einbringvorrichtung die Innenkontur des Handschuhs vollständig abdeckt.

Die Verbindung der beiden Lagen wird bevorzugt durch Schweißen und/oder als Naht gebildet.

Sowohl bei der einlagigen als auch bei der doppellagigen, handschuhförmigen Einbringvorrichtung kann das Einbringen in den zu sterilisierenden Handschuh automatisch erfolgen, bevorzugt mittels eines Roboters bzw. einer Maschine.

Beschrieben wird auch eine Einbringvorrichtung, die aus einem dampfdurchlässigen Material gebildet ist und die Kontur eines Handschuhes aufweist. Die in dem Verfahren vorteilhaften Ausgestaltungen der Einbringvorrichtung sind vorteilhafte Ausgestaltungen der Einbringvorrichtung.

Bevorzugt ist die Einbringvorrichtung einlagig ausgebildet, wobei sie bevorzugt an den Fingerspitzen Aufnahmeelemente bzw. Einführungselemente aufweist, die bevorzugt jeweils in der Form eines Fingerhutes ausgebildet sind. Der Benutzer kann die Aufnahmeelemente auf seine Fingerkuppen stülpen und dann die Einbringvorrichtung in den zu sterilisierenden Handschuh einführen. Auf diese Weise wird sichergestellt, dass die Einbringvorrichtung die gesamten Finger ausfüllt.

In einer anderen bevorzugten Ausführungsform ist die Einbringvorrichtung zweilagig ausgeführt in der Art eines Handschuhs, bei der die beiden Lagen miteinander verbunden, insbesondere verschweißt oder vernäht sind. Im Gegensatz zu bekannten sterilisierbaren Behältnissen, in denen der zu sterilisierende Gegenstand eingebracht wird oder die selbst sterilisiert werden sollen, ermöglicht die Einbringvorrichtung erst die Dampfsterilisation eines anderen Gegenstandes, nämlich eines Handschuhs, indem sie in diesen eingebracht wird, wobei die Einbringvorrichtung sowie der zu sterilisierende Gegenstand, insbesondere der Handschuh, bevorzugt als gefaltete Konfiguration vorliegen. Aus diesem Grund ist die Einbringvorrichtung vollständig aus dampfdurchlässigem Material gefertigt: der Dampf muss alle Innenflächen des zu sterilisierenden Handschuhs erreichen und sollte nicht durch dampfundurchlässige Flächen daran gehindert werden.

Beschrieben wird auch eine Sterilisationskombination mit einem Handschuh und einer oben beschrieben Einbringvorrichtung.

Beschrieben wird auch eine Sterilisationsvorrichtung mit einer Kammer für Dampfsterilisation und wenigstens einer oben beschriebenen Einbringvorrichtung.

Die Vorteile der Erfindung liegen insbesondere darin, dass eine gesicherte und zuverlässige Sterilisation eines Handschuhs, bevorzugt in gefalteter Konfiguration, bis in die Fingerspitzen ermöglicht wird. Die vorgeschlagenen Materialien sind bereits für die Dampfsterilisation zugelassen. Die Bestückung eines Handschuhs mit einer Einbringvorrichtung kann sehr schnell erfolgen, so dass der Sterilisationsvorgang sehr effektiv durchgeführt werden kann. Bei Verwendung von Aufnahmeelementen für Fingerkuppen kann auch bei einer einlagigen Einbringvorrichtung die zuverlässige Sterilisation bis in die Fingerspitzen sichergestellt werden. Bei einer als Handschuh, bzw. Innenhandschuh ausgebildeten Einbringvorrichtung kann der Benutzer besonders schnell und komfortabel die Einbringvorrichtung in den zu sterilisierenden Handschuh einbringen.

Ein Ausführungsbeispiel der Erfindung wird anhand einer Zeichnung näher erläutert. Darin zeigen in stark schematisierter Darstellung:
- FIG. 1: eine Einbringvorrichtung, die einlagig ausgebildet ist in einer bevorzugten Ausführungsform;
- FIG. 2: die Einbringvorrichtung gemäß FIG. 1 in einer vergrößerten Darstellung;
- FIG. 3: eine Einbringvorrichtung, die zweilagig ausgebildet ist, in einer bevorzugten Ausführungsform; und
- FIG. 4: einen Handschuh, der sterilisiert werden soll.

Gleiche Teile sind in allen Figuren mit denselben Bezugszeichen versehen.

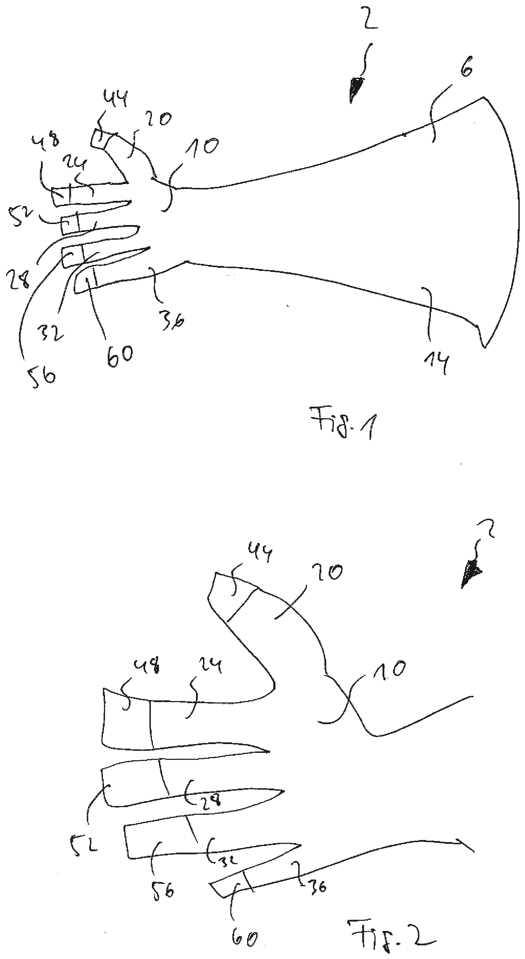

Eine in FIG. 1 dargestellte Einbringvorrichtung 2 weist einen einlagigen Grundkörper 6 auf. Der Grundkörper 6 weist einen Handbereich 10 auf sowie bevorzugt einen Unterarmbereich 14. Der Handbereich 10 weist die Kontur einer menschlichen Hand mit fünf Fingerbereichen 20, 24, 28, 32, 36 auf, wobei der Fingerbereich 20 die Kontur eines Daumens aufweist und die Fingerbereiche 24, 28, 2, 26 bevorzugt den Konturen von Zeigefinger, Mittelfinger, Ringfinger und kleinem Finger nachgebildet sind. In dem dem Unterarmteil 14 abgewandten Ende am jeweiligen Fingerbereich 20, 24, 28, 32, 36 ist am Grundkörper bevorzugt jeweils ein Führungselement 44, 48, 52, 56, 60 bzw. Aufnahmeelement angebracht, welches dazu ausgebildet ist, eine menschliche Fingerkuppe aufzunehmen. Das jeweilige Führungselement 44, 48, 52, 56, 60 ist dazu bevorzugt in der Art eines Fingerhutes ausgebildet. Es weist bevorzugt einen hohlkörperförmigen Bereich auf, welcher die Fingerkuppe des Fingers zumindest teilweise aufnimmt.

In anderen bevorzugten Ausführungsformen können auch nur ein oder weniger als fünf Führungselemente 44, 48, 52, 56, 60 vorgesehen sein. In diesem Fall kann das zumindest eine Führungselement verwendet werden, um die Einbringvorrichtung in den zu sterilisierende Handschuh zu führen und bevorzugt das entsprechende Führungselement in den Finger des Handschuhs zu führen, während die anderen Fingerbereich ohne Führungselement in den jeweiligen Finger des Handschuhs gebracht werden. In anderen bevorzugten Ausführungsformen sind keine Führungselemente vorhanden.

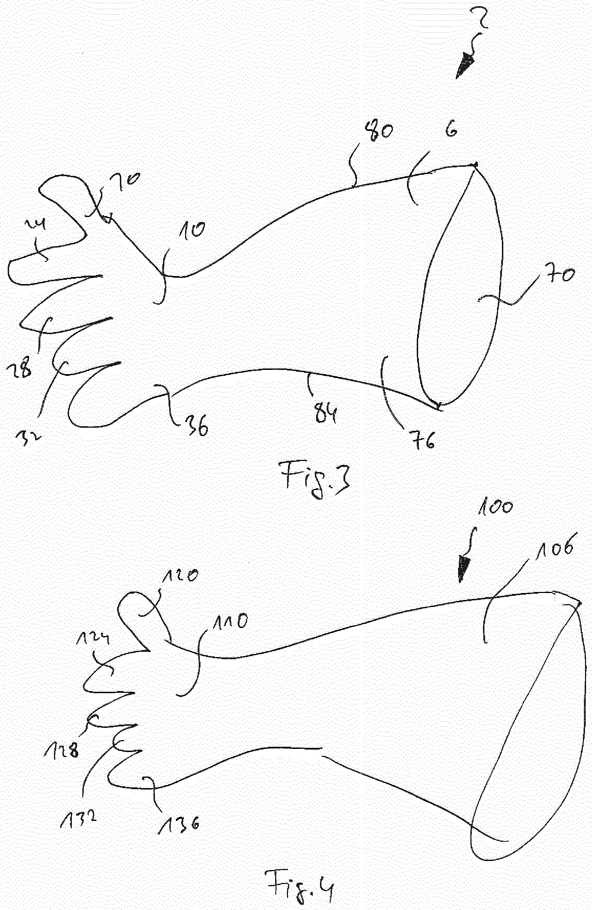

Der Grundkörper 6 und damit im Wesentlichen die Einbringvorrichtung 2 ist aus einem dampfdurchlässigen Material hergestellt, bevorzugt mindestens einlagigem dampfdurchlässigen und durch Heißdampf sterilisierbaren Kunststoffmaterial. Das Kunststoffmaterial bevorzugt aus thermisch behandeltem Fasermaterial und/oder Vliesstoff besteht. Das Fasermaterial und/oder der Vliesstoff umfasst bevorzugt Fasern von Thermoplasten oder mikrofeinem Polytetraflourethylen und/oder aus deren Mischungen, welche, insbesondere thermisch, derart behandelt wurden, dass eine Dampfdurchlässigkeit gegeben ist. Besonders bevorzugt die der Grundkörper 6 bzw. die Einbringvorrichtung 2 aus Tyvek^{®} oder porösem Kunststoffmaterial auf Polyolefinbasis hergestellt. Das jeweilige Führungselement 44, 48, 52, 56, 60 ist vorzugsweise aus dampfdurchlässigem Material gefertigt und bevorzugt aus dem gleichen Material wie der Grundkörper 6 gefertigt. Es ist mit dem Grundkörper 6 bevorzugt verbunden durch eine Siegelnaht mittels thermischer Verschweißung. Die Einbringvorrichtung 2 wird zur Sterilisation in den zu sterilisierenden Handschuh 100 (siehe FIG. 4) eingebracht, in dem die Führungselemente in die Fingerspitzen 44, 48, 52, 56, 60 des Handschuhs 100 geführt werden.

Eine Einbringvorrichtung 2 in einer zweiten bevorzugten Ausführung ist in FIG. 3 dargestellt. Der Grundkörper 6 umfasst eine erste Lage 70 und eine zweite Lage 76. Die jeweilige Lage 70, 76 weist im Wesentlichen die Kontur von Hand und Unterarm auf. Die beiden Lagen 70, 76 sind an zwei Nähten 80, 84 miteinander verschweißt. Dadurch ist die Einbringvorrichtung 2 als Handschuh ausgebildet. Sie bildet bei der Sterilisation gewissermaßen einen Innenhandschuh für den zu sterilisierenden Handschuh. Die bevorzugten Materialien für diese Ausführungsform der Einbringvorrichtung 2 sind die gleichen wie die im Zusammenhang mit der Ausführungsform gemäß FIG. 1 beschriebenen Materialien.

In FIG. 4 ist beispielhaft ein Handschuh 100, der sterilisiert werden soll, dargestellt. Der Handschuh 100 weist einen Unterarmbereich 106 und einen Handbereich 110 auf. Der Handbereich weist Fingerteile 120, 124, 128, 132, 136 auf, die in Fingerspitzen münden.

Für eine Dampfsterilisation wird ein Handschuh 100 bereitgestellt. Die in FIG. 1 dargestellte Einbringvorrichtung 2 wird in ihn eingebracht. Dazu wird die Einbringvorrichtung 2 derart in den Handschuh 100 eingebracht, dass die Fingerbereiche 20, 24, 28, 32, 26 in den Fingerbereichen 120, 124, 128, 132, 126 des Handschuhs 100 liegen. Der Unterarmbereich 14 liegt dabei möglichst deckungsgleich im Unterarmbereich 106 des Handschuhs 100.

Die Einbringvorrichtung 2 gemäß FIG. 3 wird in den Handschuh 100 eingebracht, indem die Hand in die Einbringvorrichtung 2 geführt wird und dieser auf Hand und Unterarm wie ein Handschuh getragen wird. Zusammen mit der Einbringvorrichtung 2 wird dann die Hand in den Handschuh 100 geführt, der bevorzugt währenddessen festgehalten bzw. räumlich fixiert wird, so dass die Finger in den Fingerbereichen 120, 124, 128, 132, 136 eingebracht sind. Die Hand wird dann wieder aus der Einbringvorrichtung 2 gezogen.

Der Handschuh 100 wird in einer bevorzugten Ausführungsform vor dem Einleiten von Sterilisationsdampf gefaltet. Er kann einmal oder auch mehrfach gefaltet werden. Diese Faltung ist möglich, da das Material der Einbringvorrichtung faltbar und demgemäß entsprechend flexibel ausgebildet ist.

Nachdem wenigstens ein Handschuh 100 mit eingebrachter Einbringvorrichtung 2 in der Dampfsterilisationskammer positioniert wurde bzw. in diese gelegt wurde, wird sterilisierender Dampf in die Kammer eingeleitet.

Der Handschuh 100 wird in der Sterilisationskammer bevorzugt ausgestreckt oder einfach oder mehrfach gefaltet flach liegend angeordnet. Alternativ werden die Handschuhe kassettiert, d. h. nebeneinander auf bevorzugt flache Gestelle aufgebracht, wodurch in platzsparender Weise die Anzahl der Handschuhe 100, die in der Kammer angeordnet werden können, erhöht werden kann.

Nach Anordnung der Handschuhe 100 in der Sterilisationskammer wird Dampf, bevorzugt Sattdampf im Temperaturbereich von T = 120-135 °C in die Sterilisationskammer eingeleitet.

Die dargestellten Einbringvorrichtungen 2 werden bevorzugt sowohl für linke als auch rechte Handschuhe verwendet. Die in den FIGs. gezeigten Proportionen sind nicht maßstabsgetreu und können je nach zu sterilisierendem Handschuh auch anders gewählt bzw. auch weggelassen werden. So kann beispielsweise der Unterarmbereich 14 ganz oder teilweise fehlen oder auch länger als gezeigt ausgebildet sein. Die dargestellte Erfindung kann Modifikationen und Veränderungen aufweisen die immer noch von dem erfindungsgemäßen Konzept umfasst sind. Alle Details können durch technisch äquivalente Elemente ersetzt werden. Formen, Ausgestaltungen und Materialien können an konkrete Anforderungen angepasst werden ohne den Schutzbereich der Ansprüche zu verlassen.

Ein Verfahren zur Dampfsterilisation von Handschuhen (100) soll die Kapazität erhöhen bei gleichzeitiger Zuverlässigkeit der vollständigen Sterilisation. Dazu umfasst es die Schritte
a) Bereitstellen eines Handschuhs (100);
b) Einbringen einer Einbringvorrichtung (2) in den Handschuh (100);
c) Deponieren des Handschuhs (100) mit eingebrachter Einbringvorrichtung (2) in einer Dampfsterilisationskammer;
d) Einleiten von sterilisierendem Dampf in die Dampfsterilisationskammer, wobei die Einbringvorrichtung (2) eine Kontur aufweist, die im Wesentlichen formkongruent zu einer inneren Kontur des Handschuhs (100) ist und einen Grundkörper (6) aufweist, der aus dampfdurchlässigem Material gefertigt ist, und wobei die Einbringvorrichtung (2) derart in den Handschuh (100) eingebracht wird, dass sie im Wesentlichen die gesamte innere Kontur des Handschuhs (100) einnimmt.

## Patentansprüche

1. Verfahren zur Dampfsterilisation von Handschuhen (100), umfassend die Schritte
a) Bereitstellen eines Handschuhs (100);
b) Einbringen einer Einbringvorrichtung (2) in den Handschuh (100);
c) Deponieren des Handschuhs (100) mit eingebrachter Einbringvorrichtung (2) in einer Dampfsterilisationskammer;
d) Einleiten von sterilisierendem Dampf in die Dampfsterilisationskammer, wobei die Einbringvorrichtung (2) eine Kontur aufweist, die formkongruent zu einer inneren Kontur des Handschuhs (100) ist,
**dadurch gekennzeichnet, dass** die Einbringvorrichtung (2) flexibel ausgebildet ist und einen Grundkörper (6) aufweist, der aus dampfdurchlässigem Material gefertigt ist, und wobei die Einbringvorrichtung (2) derart in den Handschuh (100) eingebracht wird, dass sie die gesamte innere Kontur des
Handschuhs (100) einnimmt, und wobei nach Einbringen der Einbringvorrichtung (2) in den Handschuh (100) der Handschuh (100) wenigstens einmal gefaltet wird, und wobei das Material aus mindestens einlagigem dampfdurchlässigen und durch Dampf sterilisierbaren Kunststoffmaterial besteht.

2. Verfahren nach Anspruch 1, wobei das Kunststoffmaterial aus thermisch behandeltem Fasermaterial und/oder Vliesstoff besteht.

3. Verfahren nach Anspruch 2, wobei das Fasermaterial und/oder der Vliesstoff Fasern von Thermoplasten oder mikrofeinem Polytetraflourethylen und/oder aus deren Mischungen umfasst, welche, insbesondere thermisch, derart behandelt wurden, dass eine Dampfdurchlässigkeit gegeben ist.

4. Verfahren nach einem der Ansprüche1 bis 3, wobei die Einbringvorrichtung (2) einlagig ausgebildet ist.

5. Verfahren nach Anspruch 4, wobei die Einbringvorrichtung (2) Einführungselemente (44, 48, 52, 56, 60) aufweist, die in die Finger des Handschuhs (100) eingeführt werden.

6. Verfahren nach einem der Ansprüche 1 bis 3, wobei die Einbringvorrichtung (2) zumindest zweilagig ausgebildet ist, wobei jede Lage (70, 76) mit zumindest einer weiteren Lage (70, 76) zumindest teilweise an einem Randbereich (80, 84) miteinander verbunden ist.

7. Verfahren nach Anspruch 6, wobei die Einbringvorrichtung (2) in der Form eines Handschuhs ausgebildet ist.

8. Verfahren nach Anspruch 7, wobei die Einbringvorrichtung (2) zweilagig ausgebildet ist und die beiden Lagen (70, 76) derart miteinander verbunden sind, dass sie einen Handschuh bilden.

9. Verfahren nach Anspruch 8, wobei die Verbindung der beiden Lagen (70, 76) durch Schweißen oder als Naht gebildet ist.

## Claims

1. A method for the steam sterilisation of gloves (100), comprising the steps
a) provision of a glove (100);
b) introduction of an insertion device (2) into the glove (100);
c) placement of the glove (100) with the introduced insertion device (2) into a steam sterilisation chamber;
d) supplying of sterilising steam into the steam sterilisation chamber, wherein the insertion device (2) has a contour that is congruent in shape to the inner contour of the glove (100),
**characterised in that** the insertion device (2) is flexible in design and comprises a basic body (6) made of a steam-permeable material, and wherein the insertion device (2) is introduced into the glove (100) in such a way that it occupies the entire inner contour of the glove (100), and wherein after introduction of the insertion device (2) into the glove (100), the glove (100) is folded at least once and wherein the material consists of a least single-layer steam-permeable synthetic material that can be sterilised by steam.

2. The method according to claim 1, wherein the synthetic material consists of thermally treated fibre material and/or non-woven material.

3. The method according to claim 2, wherein the fibre material and/or the non-woven material comprises thermoplastic or microfine polytetrafluorethylene fibres and/or mixtures thereof, which have been treated, in particular thermally, so that permeability to steam results.

4. The method according to any one of claims 1 to 3, wherein the insertion device (2) is designed in one layer.

5. The method according to claim 4, wherein the insertion device (2) comprises introducing elements (44, 48, 52, 56, 60) which are inserted into the fingers of the glove (100).

6. The method according to any one of claims 1 to 3, wherein the insertion device (2) is designed in at least two layers, wherein each layer (70, 76) is connected to at least one other layer (70, 76) at least partially in a marginal area (80, 84).

7. The method according to claim 6, wherein the insertion device (2) is designed in the shape of a glove.

8. The method according to claim 7, wherein the insertion device is designed in two layers, and the two layers (70, 76) are connected to each other in such a way that they form a glove.

9. The method according to claim 8, wherein the connection between the two layers (70, 76) is formed by welding or as a seam.

## Revendications

1. Procédé de stérilisation à la vapeur de gants (100), comprenant les étapes de
a) mise à disposition d'un gant (100) ;
b) introduction d'un dispositif d'incorporation (2) dans le gant (100) ;
c) dépôt du gant (100) avec un dispositif d'incorporation (2) introduit dans une chambre de stérilisation à la vapeur ;
d) introduction de vapeur stérilisante dans la chambre de stérilisation à la vapeur, sachant que le dispositif d'incorporation (2) comporte un profil, qui coïncide en forme avec un profil intérieur du gant (100),
**caractérisé en ce que** le dispositif d'incorporation (2) est constitué de façon flexible et comporte un corps de base (6), qui est fabriqué dans un matériau perméable à la vapeur, et sachant que le dispositif d'incorporation (2) est incorporé dans le gant (100) de telle manière qu'il adopte tout le profil intérieur du gant (100), et sachant qu'après introduction du dispositif d'incorporation (2) dans le gant (100), le gant (100) est plié au moins une fois et sachant que le matériau est composé d'au moins un matériau à base de matière plastique perméable à la vapeur monocouche et stérilisable par la vapeur.

2. Procédé selon la revendication 1, sachant que le matériau à base de matière plastique est composé de matériau de fibres et/ou de matière de non-tissé ayant subi un traitement thermique.

3. Procédé selon la revendication 2, sachant que le matériau de fibres et/ou la matière de non-tissé comprend des fibres de matières thermoplastiques ou du polytétrafluoréthylène ultrafin et/ou des mélanges de ceux-ci, lesquels ont été traités en particulier thermiquement de telle manière qu'une perméabilité à la vapeur est attribuée.

4. Procédé selon l'une quelconque des revendications 1 à 3, sachant que le dispositif d'incorporation (2) est constitué d'une couche.

5. Procédé selon la revendication 4, sachant que le dispositif d'incorporation (2) comporte des éléments d'incorporation (44, 48, 52, 56, 60), qui sont introduits dans les doigts du gant (100).

6. Procédé selon l'une quelconque des revendications 1 à 3, sachant que le dispositif d'incorporation (2) est constitué d'au moins deux couches, sachant que chaque couche (70, 76) est reliée avec au moins une autre couche (70,76) au moins en partie sur une zone de bord (80, 84).

7. Procédé selon la revendication 6, sachant que le dispositif d'incorporation (2) est constitué sous la forme d'un gant.

8. Procédé selon la revendication 7, sachant que le dispositif d'incorporation (2) est constitué de deux couches et les deux couches (70, 76) sont reliées entre elles de telle manière qu'elles forment un gant.

9. Procédé selon la revendication 8, sachant que la liaison des deux couches (70, 76) est formée par soudure ou sous la forme de couture.
